(19) Europäisches Patentamt
European Patent Office
Office européen des brevets

(11) Veröffentlichungsnummer: **0 185 257**
**A1**

# (12) EUROPÄISCHE PATENTANMELDUNG

(21) Anmeldenummer: **85115439.3**

(22) Anmeldetag: **05.12.85**

(51) Int. Cl.⁴: **C 07 D 405/12**
**A 01 N 47/22**

(30) Priorität: **18.12.84 DE 3446103**

(43) Veröffentlichungstag der Anmeldung:
**25.06.86 Patentblatt 86/26**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Heywang, Gerhard, Dr.**
**Nittumer Weg 4**
**D-5060 Bergisch-Gladbach 2(DE)**

(72) Erfinder: **Becker, Benedikt, Dr.**
**Metzkausener Strasse 14**
**D-4020 Mettmann(DE)**

(72) Erfinder: **Brandes, Wilhelm, Dr.**
**Eichendorffstrasse 3**
**D-5653 Leichlingen(DE)**

(72) Erfinder: **Homeyer, Bernhard, Dr.**
**Obere Strasse 28**
**D-5090 Leverkusen 3(DE)**

(72) Erfinder: **Reinecke, Paul, Dr.**
**Steinstrasse 8**
**D-5090 Leverkusen 3(DE)**

(54) **Azolhaltige carbamoylierte Oxamidsäureester, Verfahren zu ihrer Herstellung sowie ihre Verwendung als Pflanzenschutzmittel.**

(57) Es werden neue Oxamidsäureester der allgemeinen Formel (I)

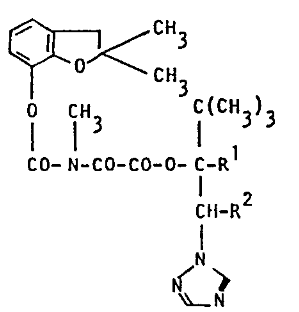

(I)

bereitgestellt,
in welcher
$R^1$ Für Wasserstoff, gegebenenfalls substituiertes Phenylethyl oder gegebenenfalls substituiertes Phenoxymethyl steht und
$R^2$ für Wasserstoff oder gegebenenfalls substituiertes Phenoxy steht,
sowie ihre Säure- oder Metallsalz-Additionsprodukte.

Die neuen Verbindungen der Formel (I) zeigen starke insektizide und fungizide Eigenschaften.



Croydon Printing Company Ltd

BAYER AKTIENGESELLSCHAFT
5090 Leverkusen, Bayerwerk
Konzernverwaltung RP
Patentabteilung
Er/Kü-c
Ia

17. DEZ. 1984

Azolhaltige carbamoylierte Oxamidsäureester, Verfahren zu ihrer Herstellung sowie ihre Verwendung als Pflanzenschutzmittel

Die vorliegende Erfindung betrifft neue azolhaltige carbamoylierte Oxamidsäureester, ein Verfahren zu ihrer Herstellung sowie ihre Verwendung als Pflanzenschutzmittel.

Es ist bereits bekannt geworden, daß bestimmte carbamoylierte 3,3-Dimethyl-1-phenoxy-1-(1,2,4-triazol-1-yl)-2-butanole, wie beispielsweise 1-(4-Biphenylyl-# oxy)-3,3-dimethyl-2-methoxycarbonylcarbamoyloxy-1-(1,2,4-triazol-1-yl)-butan oder 1-(4-Biphenylyloxy)- bzw. 1-(2,5-Dichlorphenoxy)- bzw. 1-(2,4,5-Trichlorphenoxy)-3,3-dimethyl-2-methylcarbamoyloxy-1-(1,2,4-triazol-1-yl)-butan, gute fungizide Eigenschaften besitzen (vergleiche DE-OS 2 600 799 und DE-OS 2 800 544). Die Wirkung dieser Verbindungen ist jedoch in bestimmten Indikationsbereichen, insbesondere bei niedrigen Aufwandmengen und -konzentrationen, nicht immer ganz befriedigend.

Es wurden neue azolhaltige carbamoylierte Oxamidsäureester der allgemeinen Formel (I)

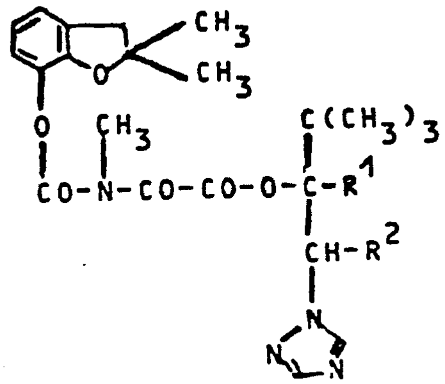

(I)

in welcher

$R^1$ für Wasserstoff, gegebenenfalls substituiertes Phenylethyl oder gegebenenfalls substituiertes Phenoxymethyl steht und

$R^2$ für Wasserstoff oder gegebenenfalls substituiertes Phenoxy steht,

gefunden.

Die Verbindungen der Formel (I) besitzen zum Teil zwei asymmetrische Kohlenstoffatome; sie können somit in diesen Fällen in zwei geometrischen Isomeren (threo- und erythro-Form) vorliegen. Vorwiegend fallen sie dann als Gemische beider Formen an.

Weiterhin wurde gefunden, daß man die neuen carbamoylierten Oxamidsäureester der Formel I erhält, wenn man 1-(1,2,4)Triazolyl-2-butanole der Formel (II)

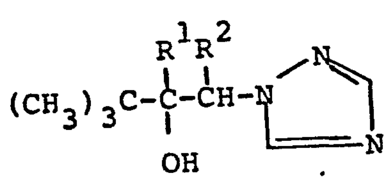

(II)

in welcher $R^1$ und $R^2$ die oben angegebene Bedeutung haben, mit N-(2,3-Dihydro-2,2-dimethyl-benzofuran-7-oxy-carbonyl)-N-methyl-2-amino-2-oxo-acetylhalogeniden der Formel (III)

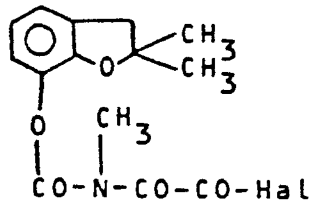

(III)

in welcher

Hal für Halogen, vorzugsweise Chlor oder Brom steht, in Gegenwart eines Verdünnungsmittel und in Gegenwart eines Säurebindemittels umsetzt.

An die so erhaltenen Verbindungen der Formel (I) kann gegebenenfalls anschließend noch eine Säure oder ein Metallsalz addiert werden.

Die neuen azolhaltigen carbamoylierten Oxamidsäureester der Formel I weisen starke insektizide und fungizide Eigenschaften auf. Dabei zeigen überraschenderweise die erfindungsgemäßen Verbindungen eine bessere insektizide und eine bessere fungizide Wirkung als die aus dem Stand der Technik bekannten carbamoylierten 3,3-Dimethyl-1-phenoxy-1-(1,2,4-triazol-1-yl)-2-butanole, wie 1-(4-Biphenylyloxy)-3,3-dimethyl-2-methoxycarbonylcarbamoyloxy-1-(1,2,4-triazol-1-yl)-butan oder 1-(4-Biphenylyloxy)- bzw. 1-(2,5-Dichlorphenoxy)- bzw.

1-(2,4,5-Trichlorphenoxy)-3,3-dimethyl-2-methylcarbamoyloxy-1-(1,2,4-triazol-1-yl)-butan, welche konstitutionell und wirkungsmäßig naheliegende Verbindungen sind. Die erfindungsgemäßen Stoffe stellen somit eine Bereicherung der Technik dar.

Die erfindungsgemäßen azolhaltigen carbamoylierten Oxamidsäureester sind durch die Formel (I) allgemein definiert.

In der Formel (I) stehen $R^1$ und $R^2$ vorzugsweise nicht gleichzeitig für Wasserstoff.

In dieser Formel stehen vorzugsweise

$R^1$ für Wasserstoff, gegebenenfalls einfach oder zweifach, gleich oder verschieden substituiertes Phenylethyl, gegebenenfalls einfach oder zweifach, gleich oder verschieden substituiertes Phenoxymethyl, wobei als Substituenten genannt seien: Halogen, Alkyl mit 1 bis 4 Kohlenstoffatomen, der Rest $-CH=NO-Alkyl(C_1-C_4)$ und gegebenenfalls durch Halogen substituiertes Phenyl, und

$R^2$ für Wasserstoff und gegebenenfalls einfach oder zweifach, gleich oder verschieden substituiertes Phenoxy, wobei als Substituenten genannt seien: Halogen, Alkyl mit 1 bis 4 Kohlenstoffatomen und gegebenenfalls durch Halogen substituiertes Phenyl.

Besonders bevorzugt sind diejenigen Verbindungen der Formel (I), in denen

$R^1$ für Wasserstoff, einfach oder zweifach, gleich oder unterschiedlich durch Fluor, Chlor, Methyl, den Rest $-CH=N-OCH_3$ oder Phenyl substituiertes Phenylethyl oder einfach oder zweifach, gleich oder unterschiedlich durch Fluor, Chlor, Methyl, den Rest $-CH=N-OCH_3$ oder Phenyl substituiertes Phenoxymethyl und

$R^2$ für Wasserstoff, einfach oder zweifach, gleich oder unterschiedlich durch Fluor, Chlor, Methyl oder Phenyl substituiertes Phenoxy steht.

Ganz besonders bevorzugt sind diejenigen Verbindungen der Formel I, in denen

$R^1$ für Wasserstoff und

$R^2$ für 4-Chlorphenoxy, 2,4-Dichlorphenoxy, 4-Methylphenoxy, 2,4-Dimethylphenoxy, 4-Phenylphenoxy steht

und diejenigen Verbindungen der Formel I, in denen

$R^1$ für 2-(4-Chlorphenyl)ethyl, 2-(2,4-Dichlorphenyl)-ethyl, 4-Chlorphenoxymethyl, 2,4-Dichlorphenoxymethyl oder den Rest $-CH_2-O-$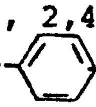$-CH=NOCH_3$ steht und

$R^2$ für Wasserstoff steht.

Verwendet man beispielsweise 5-(4-Chlorphenyl)-2,2-dimethyl-3-(1,2,4-triazol-1-yl-methyl)-3-pentanol und N-(2,3-Dihydro-2,2-dimethyl-benzofuran-7-oxy-carbonyl)-N-methyl-2-amino-2-oxo-acetylchlorid als Ausgangsstoffe, so kann der Reaktionsablauf des erfindungsgemäßen Verfahrens durch das folgende Formelschema wiedergegeben werden

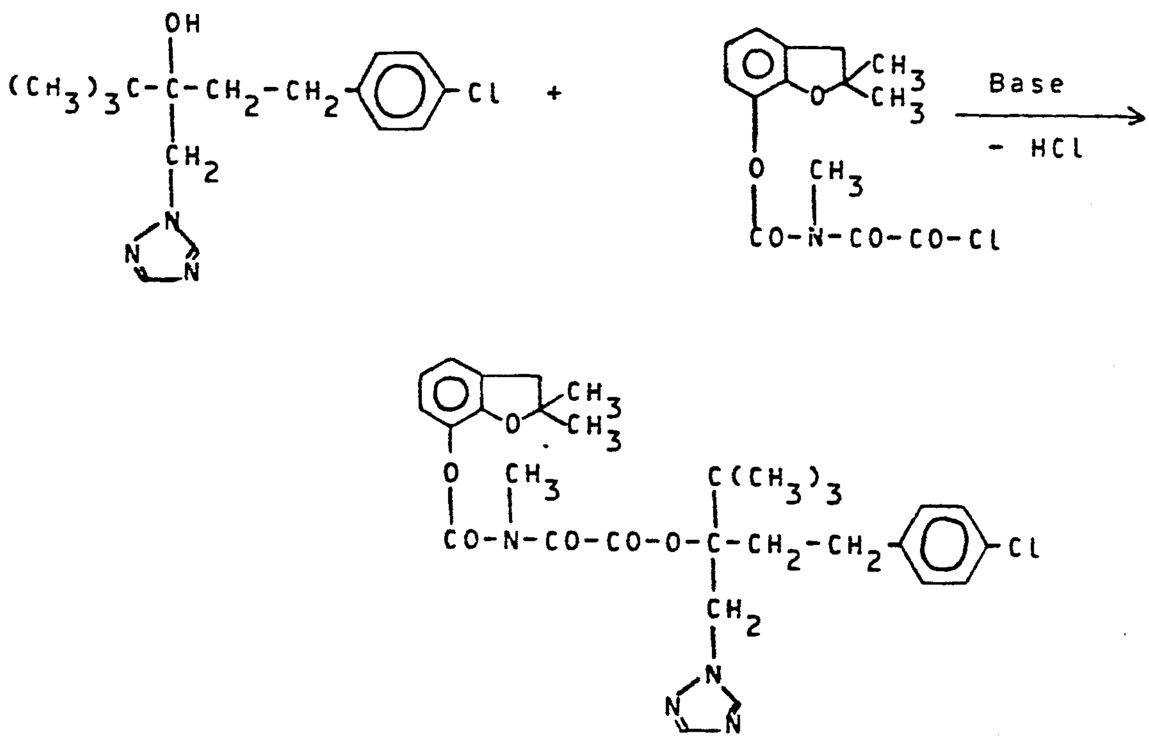


Die bei der Durchführung des erfindungsgemäßen Verfahrens als Ausgangsstoffe benötigten 1-Azolyl-3,3-dimethyl-1-phenoxy-2-butanole sind durch die Formel

(II) allgemein definiert. In dieser Formel stehen $R^1$ und $R^2$ vorzugsweise für die Bedeutungen, die bei der Beschreibung der erfindungsgemäßen Stoffe der Formel (I) bereits vorzugsweise für diese Reste genannt wurden.

Die 1-Azolyl-3,3-dimethyl-2-butanole der Formel (II), in welcher $R^1$ für Wasserstoff steht und $R^2$ von Wasserstoff verschieden ist, sind bekannt (vergleiche DE-PS 2 324 010 und DE-OS 2 333 354) und können nach den dort angegebenen Verfahren in üblicher Art und Weise erhalten werden.

Die 1-Azolyl-3,3-dimethyl-2-butanole der Formel (II), in welcher $R^1$ von Wasserstoff verschieden ist und $R^2$ für Wasserstoff steht, sind bekannt (vergleiche DOS 3 018 866 (Le A 20 330)) und können nach den dort angegebenen Verfahren in üblicher Art und Weise erhalten werden.

Das außerdem für das erfindungsgemäße Verfahren als Ausgangsstoff zu verwendende N-(2,3-Dihydro-dimethyl-benzofuran-7-oxy-carbonyl)-N-methyl-2-amino-2-oxo-acetylchlorid (Formel III mit Hal = Cl) ist ebenfalls bekannt (vergleiche dazu DE-OS 3 205 195).

Als Verdünnungsmittel kommen für das erfindungsgemäße Verfahren inerte organischen Lösungsmittel infrage. Hierzu gehören vorzugsweise aromatische Kohlenwasserstoffe, wie Benzol oder Toluol; halogenierte Kohlenwasserstoffe, wie Methylenchlorid oder Tetrachlorkohlenstoff; Nitrile, wie Acetonitril oder Propionitril; Ether wie Tetrahydrofuran oder Dioxan; sowie Ester, wie Essigsäuremethylester.

Als Säurebindemittel können für das erfindungsgemäße Verfahren alle üblichen organischen und anorganischen Säurebinder eingesetzt werden. Hierzu gehören vorzugsweise tertiäre Amine, wie Triethylamin; Alkalihydroxide und Alkalicarbonate, wie Natrium- und Kaliumhydroxid.

Die Reaktionstemperaturen können bei der Durchführung des erfindungsgemäßen Verfahrens in einem größeren Bereich variiert werden. Im allgemeinen arbeitet man zwischen 0 und 100°C, vorzugsweise zwischen 20 und 85°C.

Bei der Durchführung des erfindungsgemäßen Verfahrens arbeitet man vorzugsweise in äquimolaren Mengen. Die Isolierung der Endprodukte erfolgt in allgemein üblicher Art und Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren,wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Fungizide können die erfindungsgemäßen Wirkstoffe mit besonders gutem Erfolg zur Bekämpfung von Getreidemehltau (Erysiphe graminis), von Gurkenmehltau

(Sphaerotheca fuliginea) und von Reiskrankheiten, wie Pellicularia sasakii eingesetzt werden; weiterhin können die erfindungsgemäßen Wirkstoffe als Sproßfungizide gegen Rost, Septoria nodorum, Cochliobolus sativus und Pyrenophora teres sowie als Saatbeizmittel gegen die Streifenkrankheit der Gerste (Drechslera graminea), Schneeschimmel (Fusarium nivale) und Fusarium culmorum an Getreide eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella

germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusiani, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp.,

Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Als Insektizide können die erfindungsgemäßen Wirkstoffe mit besonders gutem Erfolg zur Bekämpfung von Doralis fabae und von Phaedon-Larven eingesetzt werden. Außerdem ist die gute bodeninsektizide und wurzelsystemische Wirkung hervorzuheben.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanze in bestimmter gewünschter Weise beeinflussen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase,wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vorgelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von

Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,2 %, am Wirkungsort erforderlich.

Auch beim Einsatz der erfindungsgemäßen Stoffe als Insektizide kann der Wirkstoffgehalt der aus den handelsüblichen Formulierungen bereiteten Anwendungsformen in weiteren Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten (mit Kalk gestrichenen) Unterlagen aus.

Die Herstellung und die Verwendung der erfindungsgemäßen Stoffe werden durch die folgenden Beispiele veranschaulicht.

Beispiel 1

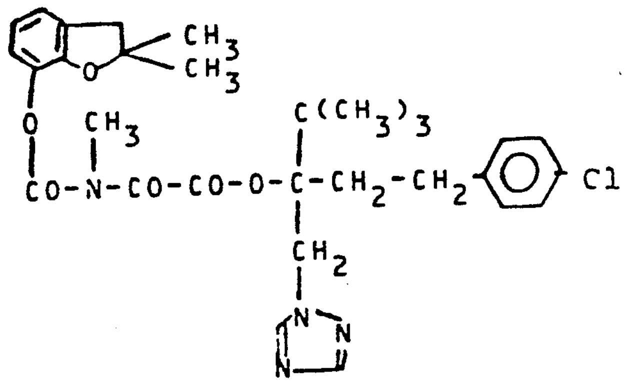

10,1 g (0,033 Mol) 5-(4-Chlorphenyl-2,2-dimethyl-3-(1,2,4-triazol-1-ylmethyl)-3-pentanol werden in 200 ml Toluol vorgelegt und mit 10,3 g (0,033 mol) N-(2,3-Dihydro-2,2-dimethyl-benzofuran-7-oxy-carbonyl)-N-methyl-2-amino-2-oxo-acetylchlorid in 50 ml Toluol gelöst versetzt.

Unter Rühren werden bei Raumtemperatur 4,6 ml (0,033 Mol) Triethylamin zugetropft. Anschließend wird das Reaktionsgemisch 2 Stunden auf 40°C erwärmt, nach dem Abkühlen mit $H_2O$ gewaschen, über Magnesiumsulfat getrocknet und vom Lösungsmittel befreit: Man erhält 10,7 g eines zähen Öls, das in Diisopropylether aufgenommen wird. Der auskristallisierte Feststoff wird abfiltriert und verworfen (Edukt). Nach Abdestillieren des Diisopropylethers verbleiben 7 g öliges Produkt (35 % der Theorie).

Analog werden hergestellt:

Beispiel 2

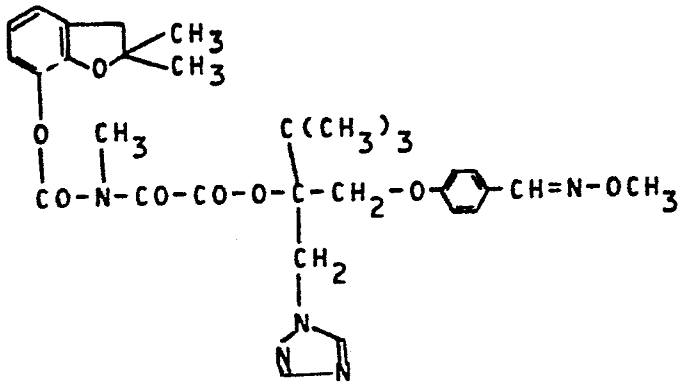

Ausbeute: 23 %, zähes Öl.

Beispiel 3

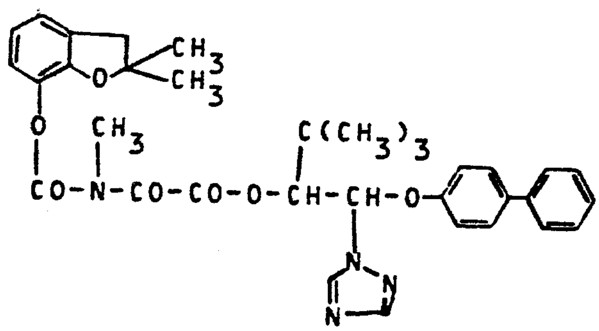

Ausbeute 30 %.

Fp. 208 - 214°C

Verwendungsbeispiele

In den nachfolgenden Beispielen werden die nachstehend angegebenen Verbindungen als Vergleichssubstanzen eingesetzt:

a) 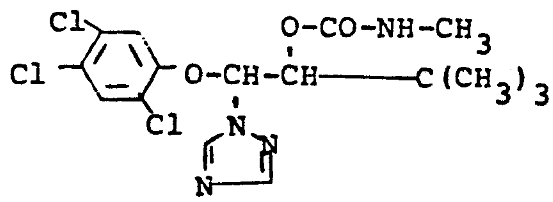

b) 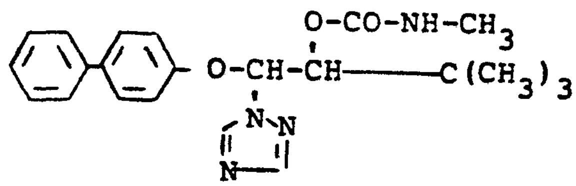

c) 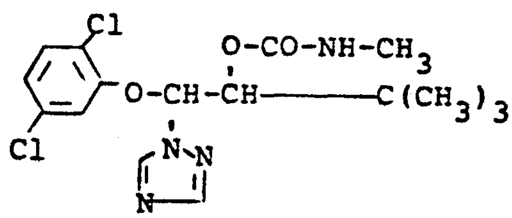

d) 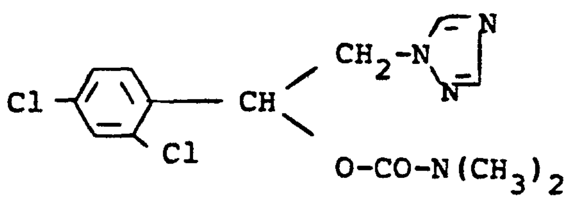

e) 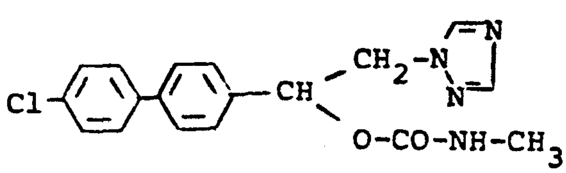

Beispiel A

Puccinia-Test (Weizen) / protektiv /

| | | | |
|---|---|---|---|
| Lösungsmittel: | 100 | Gewichtsteile | Dimethylformamid |
| Emulgator: | 0,25 | Gewichtsteile | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 2.

Beispiel B

Drechslera graminea-Test (Gerste) / Saatgutbehandlung
(syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäß folgender Herstellungsbeispiele: 1, 2.

## Beispiel C

Uromyces-Test (Buschbohne) / protektiv

Lösungsmittel: 4,7 Gewichtsteile Aceton
Emulgator: 0,3 Gewichtsteile Alkyl-aryl-polyglycolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22°C und 100 % relativer Luftfeuchtigkeit.

Die Pflanzen werden dann unter intensiver Belichtung für 9 Tage bei 20 bis 22°C und einer relativen Luftfeuchtigkeit von 70 bis 80 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 2.

**Beispiel D**

Plutella-Test

Lösungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 3.

Beispiel E

Doralis-Test (systemische Wirkung)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1, 2, 3.

**Beispiel F**

Grenzkonzentrations-Test / Bodeninsekten

Testinsekt: Phorbia antiqua-Maden im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirksamkeit gegenüber dem Stand der Technik: 1, 2, 3.

Beispiel G

Grenzkonzentrations-Test

| | |
|---|---|
| Testnematode: | Globodera rostochiensis |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 20°C.

Nach vier Wochen werden die Wurzeln auf Nematodenbefall untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise versuchtem Boden.

Bei diesem Test zeigen z.B. die folgenden Verbindungen der Herstellungsbeispiele überlegene Wirksamkeit gegenüber dem Stand der Technik: 1, 2, 3.

**Beispiel H**

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3.

Patentansprüche

1. Azolhaltige carbamoylierte Oxamidsäureester der allgemeinen Formel

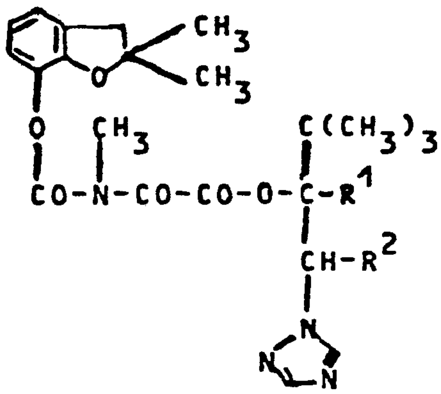

(I)

in welcher

$R^1$ für Wasserstoff, gegebenenfalls substituiertes Phenylethyl oder gegebenenfalls substituiertes Phenoxymethyl steht und

$R^2$ für Wasserstoff oder gegebenenfalls substituiertes Phenoxy steht,

sowie ihre Säure- oder Metallsalz-Additionsprodukte.

2. Verbindungen der Formel (I) in Anspruch 1, wobei

$R^1$ für Wasserstoff, einfach oder zweifach, gleich oder unterschiedlich durch Fluor, Chlor, Methyl, den Rest $-CH=NOCH_3$ oder Phenyl substituiertes

Phenylethyl oder für einfach oder zweifach, gleich oder unterschiedlich durch Fluor, Chlor, Methyl, den Rest $-CH=NOCH_3$ oder Phenyl substituiertes Phenoxymethyl steht und

$R^2$ für Wasserstoff, einfach oder zweifach, gleich oder unterschiedlich durch Fluor, Chlor, Methyl oder Phenyl substituiertes Phenoxy steht.

3. Verfahren zur Herstellung von carbamoylierten Oxamidsäureestern der allgemeinen Formel (I)

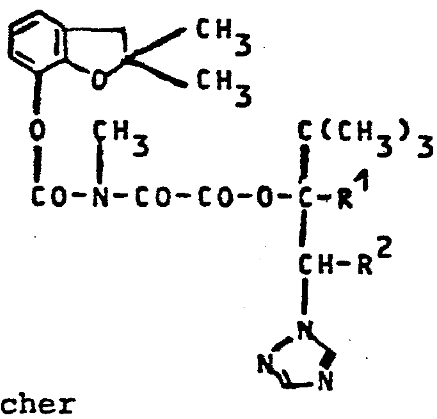

(I)

in welcher

$R^1$ für Wasserstoff, gegebenenfalls substituiertes Phenylethyl oder gegebenenfalls substituiertes Phenoxymethyl steht und

$R^2$ für Wasserstoff oder gegebenenfalls substituiertes Phenoxy steht,

dadurch gekennzeichnet, daß man 1-(1,2,4)-Triazolyl-2-butanole der Formel (II)

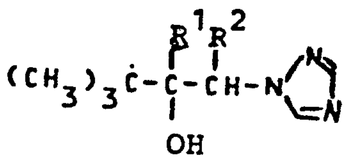

(II)

in welcher

$R^1$ und $R^2$ die oben angegebene Bedeutung haben, mit N-(2,3-Dihydro-2,2-dimethyl-benzofuran-7-oxy-carbonyl)-N-methyl-2-amino-2-oxo-acetylhalogeniden der Formel

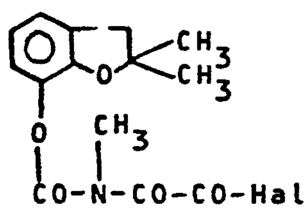

(III)

in welcher

Hal für Halogen steht, in Gegenwart eines Verdünnungsmittels und in Gegenwart eines Säurebindemittels umsetzt.

4. Insektizide und fungizide Mittel, gekennzeichnet durch einen Gehalt an mindestens einem carbamoylierten Oxamidsäureester der Formel (I) in Ansprüchen 1 und 3.

Europäisches Patentamt

# EUROPÄISCHER RECHERCHENBERICHT

0185257
Nummer der Anmeldung

EP 85115439.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | EP - A1 - 0 044 994 (BAYER)<br>* Formel I, Ansprüche 2,5 *<br>-- | 1,3,4 | C 07 D 405/12<br>A 01 N 47/22 |
| A | DE - A1 - 3 138 702 (CIBA-GEIGY)<br>* Zusammenfassung *<br>-- | 1,4 | |
| A | DE - A1 - 3 139 250 (BASF)<br>* Formel I *<br>---- | 1,4 | |

RECHERCHIERTE SACHGEBIETE (Int. Cl 4)

C 07 D 405/00

Der vorliegende Recherchenbericht wurde für alle Patentansprüche erstellt

| Recherchenort | Abschlußdatum der Recherche | Prüfer |
|---|---|---|
| WIEN | 21-02-1986 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument